# EUROPEAN PATENT SPECIFICATION

(11) **EP 0 515 406 B1**
(45) Date of publication and mention of the grant of the patent: **27.12.1995**
(21) Application number: 91903204.5
(22) Date of filing: 15.02.1991
(51) Int. Cl.: C12N 1/18

(54) **A NOVEL HIGH DENSITY YEAST PREPARATION, A METHOD FOR PRODUCING THE SAME, AND THE USE THEREOF**
EIN NEUES HOCHDICHTES HEFEPRÄPARAT, METHODE ZU DESSEN HERSTELLUNG UND DESSEN VERWENDUNG
NOUVELLE PREPARATION DE LEVURE HAUTE-DENSITE, PROCEDE DE PRODUCTION ET POSSIBILITES D'USAGE ASSOCIES

(30) Priority: 16.02.1990 FI 900804
(43) Date of publication of application: 02.12.1992
(73) Proprietor: Alko Group Ltd., 00180 Helsinki (FI)
(72) Inventor: Suoranta, Kari, SF-02210 Espoo (FI)
(74) Representative: Gaunt, Robert John
(86) International application number: PCT/FI91/00047
(87) International publication number: WO 91/12315

(56) References cited:
- EP-A- 0 259 739
- DE-C- 3 625 170
- US-A- 0 028 276
- US-A- 3 960 664
- US-A- 4 226 940
- US-A- 4 719 114

## Description

The present invention relates to a novel high-density liquid or pasty yeast preparation which contains a polyhydroxy compound, preferably glycerol. The invention also relates to a method for producing said preparation as well as the use of the same.

Nowadays baker's yeast is usually supplied for households in the form of fresh compressed yeast (in packages) or in powder form (active dry yeast). Even small and medium-sized bakeries receive their yeast in the form of fresh compressed yeast or use dry yeast. A liquid yeast, also called "cream yeast" or "yeast milk", i.e. an aqueous suspension of fresh yeast in which the content of yeast (27-29% dry matter) is approximately 750 grams per 1 liter of the product, is supplied to the greatest bakeries. The liquid yeast is prepared by centrifuging (by using separators) and the maximum yeast content of the product is determined by the separating effect of the centrifuging device (separator) used.

None of the above-mentioned forms in which yeast is supplied is fully satisfactory. The time elapsing from delivery to sale and use is often rather long, and in households the need of yeast cannot be planned in advance. Therefore, fresh yeast may not be available - the cake of yeast possibly found in the refrigerator may have become dry, moldy or inactive. A dry yeast product remains, in principle, active for a long time but, in any case, it must be "awakened" properly before use to recover full activity.

What was said above regarding dry yeast products applies also to small and medium-sized bakeries. With respect to fresh compressed yeast, the problems include the assessment of the necessary stock and the batching and crumbling of yeast, which is difficult when automatic equipment is used.

The liquid yeast product supplied to large bakeries has a tendency to sediment on the bottom of the container in which it is transported. Consequently, the yeast cream has to be stirred before use. Finally, one cubic meter of such a product contains more than 200 liters of loose, excess water.

Thus the present invention aims at eliminating the disadvantages described above.

One of the objects of the invention is to provide a novel, easily batched, high-density, liquid or pasty yeast preparation.

Another object is to provide a yeast preparation which is easily dissolved (suspendable).

A further object of the invention is to provide a liquid yeast preparation the density of which can be regulated to produce preparations of desired yeast density.

Yet another object is to provide a yeast preparation which has an improved ability to tolerate freezing temperatures, i.e. to tolerate repeated thawing and refreezing.

It is also an object of the invention to provide a liquid yeast preparation which does not sediment and thus does not require stirring before use.

The above-mentioned objects are achieved by the novel method for producing a novel high-density yeast preparation.

The novel preparation contains yeast and a polyhydroxy compound. Preferred polyhydroxy compounds are propylene glycol, glycerol, non-fermentable mono- or oligosaccharides, such as xylose, or non-fermentable sugar alcohols, such as mannitol and sorbitol, soluble oligo- or polymeric carbohydrates such as partially hydrolysed starch, cellulose or agarose and polyethylene glycol or mixtures thereof. Glycerol is an especially advantageous additive for the production of a yeast preparation, because it is an approved food additive, which can be used e.g. as a stabilizer in "coffee-bread" such as buns, scones and cinnamon rolls. In a preferred embodiment of the invention the preparation contains 10-200 g glycerol per 1 kg of fresh compressed yeast (27-29% dry matter). The concentration of the polyhydroxy compound in the extracellular liquid is 5-50% (w/v).

The novel preparation contains more than 800 g yeast (27-29% dry matter) per 1 liter of the liquid or pasty preparation. The pasty preparation according to the present invention may contain up to 1100 g yeast per 1 liter of the preparation. The liquid preparation typically contains 800 to 1050 g yeast per 1 liter of the preparation and is further characterized by low viscosity, typically less than 200 cP (20°C).

The novel high-density liquid yeast preparation is e.g. useful as a substitute for "cream yeast" and improves the properties of both "conventional" baker's yeast and baker's special yeasts such as sourdough yeast as well as those of brewer's yeast, distiller's yeast and wine yeasts. The novel high-density, liquid or pasty, yeast preparations are easy to batch and they dissolve instantly.

Figure 1 depicts the effect of freezing-thawing-refreezing on the activity of a glycerol yeast preparation (two samples, five freezing-thawing cycles).

Figure 2 depicts the effect of the time of storage at -20°C on the activity of a glycerol yeast preparation.

Figure 3 depicts the liquefaction of yeast with glycerol.

Figures 4 and 5 depict the effect of different polyols on the viscosity of a liquid yeast preparation.

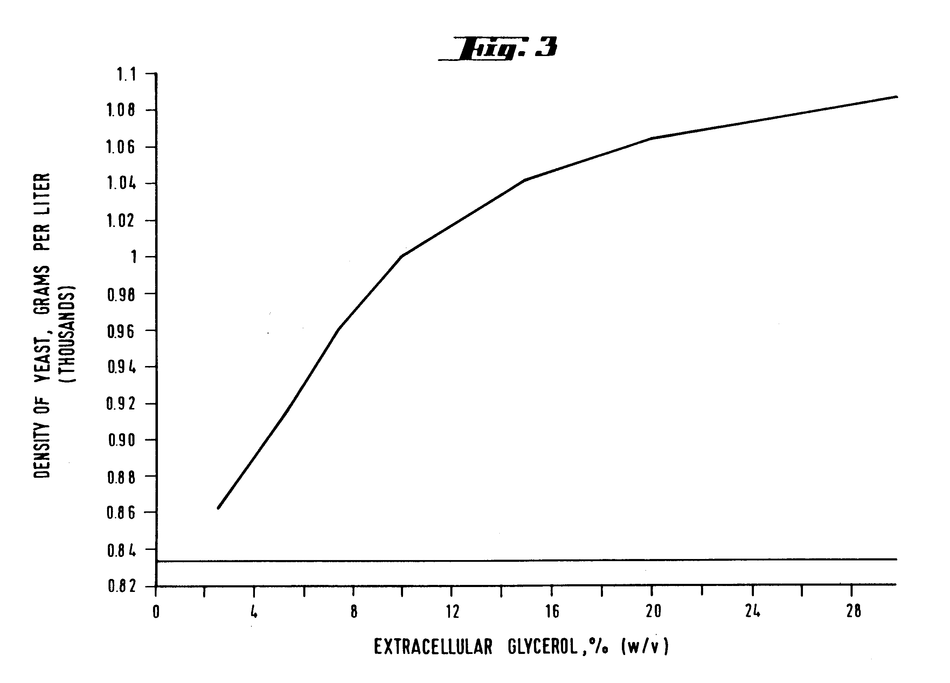

The invention is based on the quite unexpected observation that an extremely small amount of a polyhydroxy compound, preferably glycerol makes the compressed yeast "melt" or become liquefied. Figure 3 shows the liquefaction of yeast by adding glycerol. It was also found that the volume of a mixture of compressed yeast and glycerol or another polyhydroxy compound may be even smaller than the volume of the compressed yeast before the addition of said compound.

The viscosity and the volume of the preparation is a function of both the yeast content and the extracellular concentration of the polyhydroxy compound. The curve in Figure 3 indicates the approximate borderline between the liquid and the pasty forms: the area above the curve indicates the range where the preparation tends to be a paste, and the area below the curve indicates the range where the preparation is readily flowing. The horizontal line at 830 grams per liter shows the maximum density of conventional liquid yeast products.

It is well known that polyhydroxy compounds, such as glycol and glycerol, prevent freezing and even in the event of freezing they protect proteins from denaturation. Polyhydroxy compounds are also used as auxiliary agents in laboratory conditions in the storing of bacteria and yeasts (Minicool process, -70°C). Storage as well as stabilization with glycerol are previously known methods for storing bacteria and yeast strains (US 4,226,940; US 3,960,664; US Re 28,276; EP 259 739; and GB 1 530 866).

The liquefaction of yeast by adding polyhydroxy compounds, preferably glycerol, has not been previously disclosed or applied industrially. The liquefaction is based on the fact that commercial yeast contains approximately 72% water, of which 70% is intracellular and the rest extracellular (Oura, E. & Tanner, R., Changes in the Content of External and Internal Water in Pressed Yeast during Storage, Int. Ferment. Symp. Yeasts, 5th, London, Ont., Canada 1980, Abstracts, p 213). Thus, approximately 20% of the weight of fresh yeast is extracellular water. When 10% by weight of glycerol is added to the yeast, a preparation is obtained wherein the extracellular liquid contains about 30% by weight of glycerol, and when 1% by weight of glycerol is added, the extracellular liquid contains about 5% by weight of glycerol (see Table 1).

**Table 1**

| The amount of glycerol to be added to compressed yeast (27-29% dry matter) and the extracellular glycerol content of the preparation obtained. | |
|---|---|
| Glycerol addition g/100 g | Extracellular glycerol % |
| 10.00 | 31.6 |
| 9.25 | 30.6 |
| 5.40 | 20.0 |
| 3.80 | 15.0 |
| 2.40 | 10.0 |
| 1.13 | 5.0 |

Preferably, the density of the extracellular liquid is adjusted to correspond to the density of the yeast cells, whereby the sedimentation typical of the conventional, aqueous "cream yeast" is avoided.

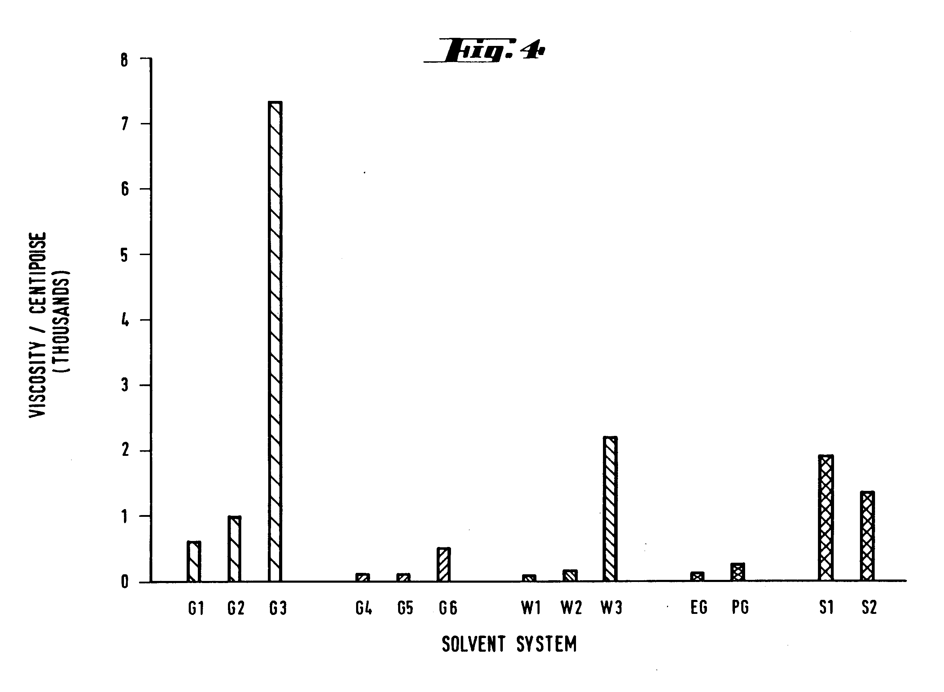

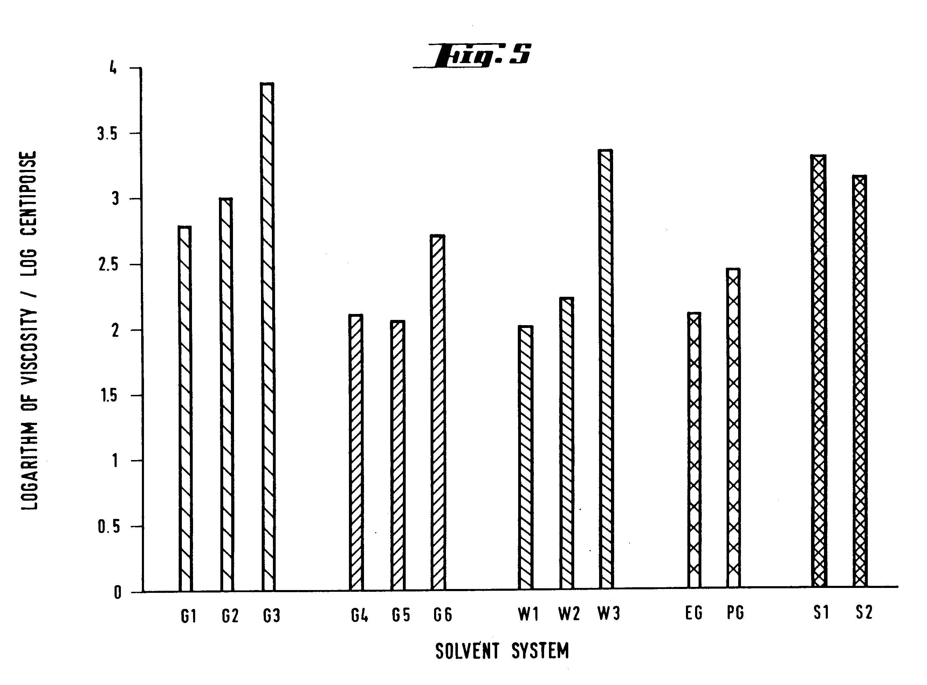

The viscosity of the liquid or pasty high-density yeast preparation according to the invention is low, and so the suspension corresponding in its yeast content, i.e. cells per liter, to compressed yeast can be poured and pumped like a liquid. The viscosity of a high-density liquid yeast preparation is typically less than 200 centiPoise (cP) at 20°C. The viscosities of conventional liquid yeasts and of different yeast preparations according to the invention are shown in Figures 4 and 5, wherein
bars G1, G2 and G3 indicate the viscosities of high-density yeast preparations that contain 1040 grams yeast (27-29% dry matter) per liter, the extracellular concentration of glycerol being 14.1, 10.6 and 7.0% (w/v), respectively, and
bars G4, G5 and G6 indicate the viscosities of high-density yeasts that have been adjusted to contain 1000 grams yeast (27-29% dry matter) per liter, the extracellular concentration of glycerol being 14.9, 11.1 and 7.4% (w/v), respectively, and
bars W1, W2 and W3 indicate the viscosities of conventional aqueous yeast products having a yeast content of 750, 800 and 830 grams per liter, respectively, and
bars EG and PG indicate the viscosities of high-density yeasts that contain 1040 grams of yeast per liter, the extracellular concentration of ethylene glycol and propylene glycol being 17.8 and 16.6% (w/v), respectively, and
bars S1 and S2 indicate the viscosities of high-density yeasts that contain 1000 and 960 grams yeast, the extracellular concentration of sorbitol being 8.3 and 10.9% (w/v), respectively.

Figure 5 shows the logarithms of the viscosities in Figure 4.

The high-density yeast preparation according to the invention contains 1-20% (w/w) of a polyhydroxy compound selected from a group comprising propylene glycol, glycerol, non-fermentable mono- or oligosaccharides or sugar alcohols, soluble oligo- or polymeric carbohydrates and polyethylene glycol, or mixtures thereof, and 80-99% (w/w) fresh yeast (27-29% dry matter).

Glycerol is the preferred polyhydroxy compound; the concentration of the glycerol solution used is preferably 60 to 90% (w/v).

Solid polyhydroxy compounds such as xylose and sorbitol have to be dissolved before use. They are typically used as 20-80% (w/v) solutions.
The preferred proportions are:
10-100 ml of 30-100% glycerol per 1 kg of fresh yeast;
60-90 ml of 30-70% (w/v) xylose per 1 kg of fresh yeast;
60-120 ml of 30-50% (w/v) sorbitol per 1 kg of fresh yeast; and
100-250 ml of 30-50% (w/v) soluble starch per 1 kg of fresh yeast.

Soluble polysaccharides such as Zulkowsky's starch are useful but may cause minor problems because they may contain fermentable sugars, which make the yeast foam during preparation.

The high-density yeast preparation according to the invention is a liquid or paste, and in a preferred embodiment it contains 10-200 g glycerol per 1 kg of fresh compressed yeast, the glycerol concentration in the extracellular liquid being 5-50%. The preparation tolerates freezing temperatures and repeated freezing, thawing and refreezing. The liquid high-density preparation has a low viscosity, i.e. less than 200 centiPoise (cP) at 20°C, so that the preparation is easy to batch and it dissolves instantly. Preferably, the density of the extracellular liquid of the preparation approaches the density of the yeast cells, whereby the sedimentation of the yeast cells is reduced and the preparation is advantageous for many industrial purposes.

The high-density yeast preparation is characterized by having a density of more than 800 g yeast per 1 liter of the yeast preparation, the concentration of the polyhydroxy compound, preferably glycerol, in the extracellular liquid being 5-50% (w/v).

This preparation has a capability of retaining its activity, dissolving instantly and of being easily batched, uniformly suspendable and tolerant of repeated freezing and thawing.

In its liquid form the high-density yeast preparation contains 3-20% (w/w) of polyhydroxy compounds selected from a group comprising propylene glycol, glycerol, non-fermentable mono- or oligosaccharides such as xylose, or non-fermentable sugar alcohols such as mannitol and sorbitol, soluble oligo- or polymeric carbohydrates such as partially hydrolysed starch, cellulose or agarose and polyethylene glycol or mixtures thereof and 80-97% of fresh yeast. In the preferred embodiment it contains 30-200 g glycerol per 1 kg of fresh compressed yeast. Said high-density liquid yeast preparation has a typical yeast content of 830-1050 g yeast (27-29% dry matter) per 1 liter of the preparation, the concentration of the polyhydrous compound in the extracellular liquid being 10-50, preferably 20% (w/v). This preparation typically has a viscosity of less than 200 centiPoise (cP) at 20°C.

In its paste form the high-density yeast preparation contains about 1-4% (w/w) of a polyhydroxy compound selected from a group comprising propylene glycol, glycerol, non-fermentable mono- or oligosaccharides such as xylose, or non-fermentable sugar alcohols such as mannitol and sorbitol, soluble oligo- or polymeric carbohydrates such as partially hydrolysed starch, cellulose or agarose and polyethylene glycol or mixtures thereof and about 96-99% (w/w) of fresh compressed yeast. In the preferred embodiment it contains 10-40 g glycerol per 1 kg of fresh compressed yeast. Said pasty high-density yeast preparation normally has a yeast content of more than 800 grams of yeast (27-29% dry matter) per 1 liter of the yeast preparation, the concentration of the polyhydroxy compound in the extracellular liquid being 5-15, preferably 10% (w/v).

Shifting to the use of the yeast preparation according to the invention will not cause batching problems for the user, since the volume of the yeast preparation can easily be adjusted so that 1 ml of the yeast preparation corresponds to 1 g of fresh yeast.

The high-density liquid or pasty yeast preparation according to our invention is prepared by adding 1-20% (w/w) of a polyhydroxy compound selected from a group comprising propylene glycol, glycerol, non-fermentable mono- or oligosaccharides such as xylose, or non-fermentable sugar alcohols such as mannitol and sorbitol, soluble oligo- or polymeric carbohydrates such as partially hydrolysed starch, cellulose or agarose and polyethylene glycol or mixtures thereof to fresh yeast by means of continuous gentle agitation. In the preferred embodiment of the invention 10-200 g glycerol is added to one kilogram of a yeast cake. When the concentration of the polyhydroxy compound, preferably glycerol, increases, the viscosity of the yeast suspension decreases sharply, i.e. the yeast becomes fluid.

If a high-density liquid yeast preparation is desired, 3-20% (w/w) of a polyhydroxy compound selected from a group comprising propylene glycol, glycerol, non-fermentable mono- or oligosaccharides such as xylose, or non-fermentable sugar alcohols such as mannitol and sorbitol, soluble oligo- or polymeric carbohydrates such as partially hydrolysed starch, cellulose or agarose and polyethylene glycol or mixtures thereof are added to fresh compressed yeast by means of gentle agitation. In a preferred embodiment of the invention 30-200 g glycerol per 1 kg fresh yeast is added by means of gentle agitation.

If a high-density pasty yeast preparation is desired, 1-4% (w/w) of a polyhydroxy compound selected from a group comprising propylene glycol, glycerol, non-fermentable mono- or oligosaccharides such as xylose, or non-fermentable sugar alcohols such as mannitol and sorbitol, soluble oligo- or polymeric carbohydrates such as partially hydrolysed starch, cellulose or agarose and polyethylene glycol or mixtures thereof is added to fresh compressed yeast by means of continuous gentle agitation. In a preferred embodiment of the invention 10-40 g glycerol per 1 kg fresh yeast is added.

The high-density liquid yeast preparation substituting for cream yeast is administered in large containers for large bakeries.

The high-density liquid yeast preparation substituting for fresh yeast is distributed either in a single consumer package containing more than one dose, optionally provided with a dosaging device such as an automatic batcher, or in a combination package comprising a plurality of single-batch containers.

The high-density pasty yeast preparation is preferably used in extrudable batching forms, such as tubes, or in squeezable capsules or metal foil or plastic bags containing one dose per package. Alternatively, it is conceivable that the preparation could be packed in small containers, in the same manner as margarine.

In addition, the invention is advantageous in the preparation of baker's special high-density yeasts such as sourdough yeast with improved quality, and it is also useful for the preparation of improved high-density brewer's yeast and wine yeasts as well.

The yeast preparation according to the invention is characterized in that in both of its forms it mixes with the dough without being pre-mixed or "awakened".

The yeast preparation according to the invention is preserved at a freezing temperature better than a yeast preparation which contains no polyhydroxy compound, preferably glycerol. The thawing of the yeast preparation is also easier due to the smaller amount of energy, only about 50%, needed for the phase transition "solid to liquid" as compared with the thawing of a (frozen) conventional liquid yeast.

In addition, the yeast preparation according to the invention can be thawed and frozen several times without that its activity decreases in a disturbing degree.

In its frozen form the yeast according to the invention is suitable for use not only in households but also in small and medium-sized bakeries.

Owing to its liquid consistence, the yeast preparation mixes easily and can be batched with automatic equipment. Frozen storage will ensure that the quality will remain even. When the yeast is stored in an air-tight container and frozen, drying as well as mold and bacterial contamination and growth are avoided.

The embodiments of the present invention are described in greater detail in the following examples.

### Example 1

### The preparation of a high-density liquid yeast

A liquid yeast preparation which flows very well is prepared by suspending 1 kg of fresh yeast in 94 g of 99.5% glycerol of USP (US Pharmacopea) quality. The yeast is added in small batches while constantly stirring the suspension gently. The preparation is suitable both for a batch package and for a package which can be used several times (thawing it before use and freezing it after use).

The glycerol concentration in the extracellular liquid is about 30% (w/v).

### Example 2

### The preparation of a high-density pasty yeast

A pasty yeast preparation is prepared by suspending 1 kg of fresh yeast in 25 g of 99.5% glycerol of USP quality. The yeast is added in small batches while constantly stirring the suspension gently. The preparation is suitable for batch packaging.

The glycerol concentration in the extracellular liquid is about 10% (w/v).

### Example 3

### The effect of storage at a low temperature on the activity of the yeast preparation

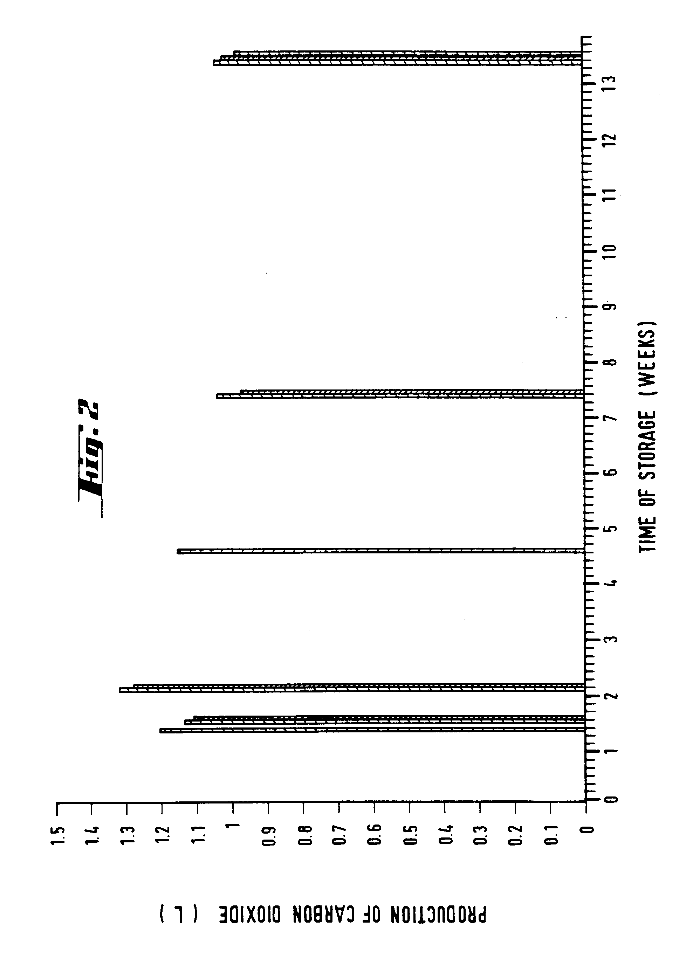

The yeast preparation prepared in Example 2 was frozen in batches of 5 g, which were stored at -20°C for 1-13 weeks. The batch to be investigated was dissolved in water at 30°C, and its activity was determined by means of a standardized baking method in which 280 g of wheat flour, 170 ml of water, 4.0 g of table salt and 5.0 ml of the yeast preparation (corresponding to 5.0 g of fresh yeast) were used for the dough. The dough was allowed to rise in a SJA fermentograph (S. J. A., Stockholm, Sweden) at 35°C under standardized conditions. The dough was allowed to rise for one hour, whereafter it was patted down and allowed to rise once more for one hour. The production of carbon dioxide per 2 hours was measured. The results are shown in Figure 2 by using two parallel samples (1) and (2).

### Example 4

### The effect of thawing-freezing cycles on the activity of the yeast preparation

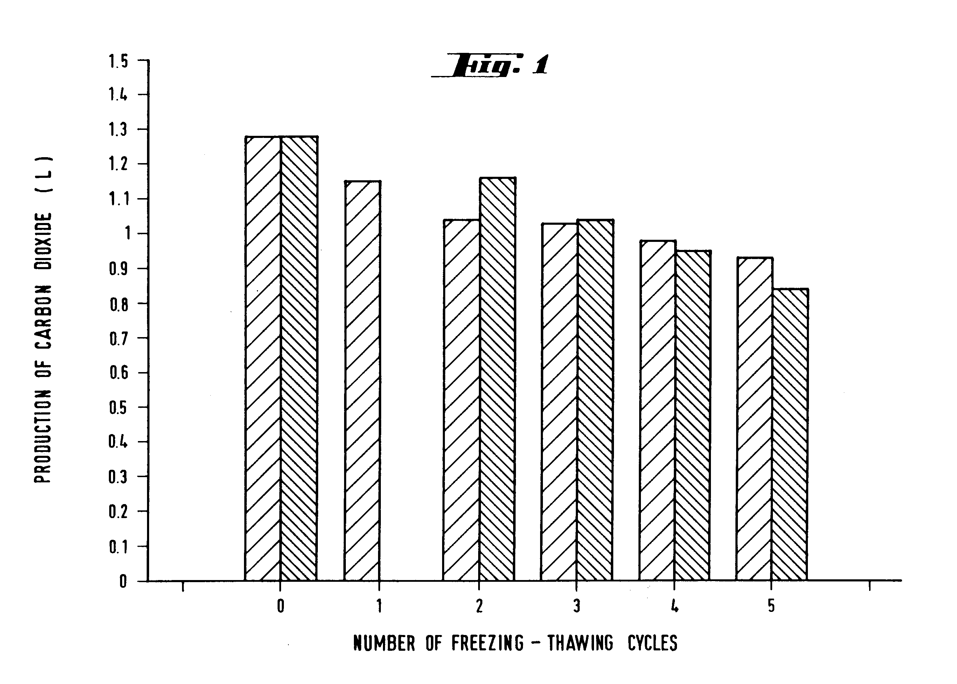

The effect of repeated thawings and freezings on the yeast prepared in Example 2 was investigated during five cycles, the duration of each cycle being 1 week. The preparation was allowed to thaw at room temperature or in water (30°C) before the measuring of the activity, and thereafter it was kept again in the freezer (-20°C) for a week. The activity was determined by means of the standardized baking method described in Example 3. The results are shown in Figure 1 by using two parallel samples (1) and (2).

### Example 5

### A brewer's yeast preparation

A preparation corresponding to the ones described in Examples 1 and 2 was prepared using a typical brewer's yeast, e.g. Saccharomyces uvarum or S. carlsbergensis, instead of ordinary fresh yeast. The typical user of such a preparation is a brewery which does not itself wish to produce the yeast it needs. The brewery can, of course, itself produce its yeast by the method according to the invention. It is advantageous to use the method or preparation according to this invention if the brewery is small and yeast is needed infrequently. A product of uniform quality is obtained by using the method and preparation according to the invention.

### Example 6

### A sourdough yeast preparation

Sourdough yeast is a traditional mixture that consists of a suitable yeast, e.g. Saccharomyces cerevisiae or Candida milleri, and suitable bacteria of the genus Lactobacillus. There are few commercial products available. Bakers traditionally use their own sourdoughs for many generations. In such a case the ending of the activity of the sourdough is a limiting factor. By the method according to the invention it is also possible to produce in a highly controlled manner a mixed suspension of yeast and suitable bacteria, the properties of which remain stable even during long-time storage in bakeries and which can be offered for retail sale in the manner described in Examples 1 and 2.

### Example 7

### A wine yeast preparation

Wine yeast is usually delivered as an active dry yeast powder to the consumers. What has been said about breweries applies also to wineries. A good preparation can be cultivated and compressed in the ordinary way.

### Example 8

### The preparation of high-density yeast containing xylose

A high-density liquid yeast preparation was made by suspending 1 kg of fresh yeast (27-29% dry matter) into 90 milliliters of a 70% (w/v) aqueous solution of xylose and mixing gently to give a homogeneous suspension. The yeast content of the product is 1000 grams per liter and the extracellular concentration of xylose is 23% (w/v).

### Example 9

### The preparation of high-density yeast containing sorbitol

High-density liquid or pasty yeast preparations were made be suspending 1 kg of fresh yeast (27-29% dry matter) into either 80 ml or 120 ml of a 30% (w/v) aqueous sorbitol solution and mixing gently. The extracellular sorbitol concentration was then 8.3 or 10.9% (w/v), respectively, and the yeast content 1000 or 960 grams per liter, respectively.

### Example 10

### The high-density liquid yeast preparation containing soluble starch

A high-density liquid yeast preparation was made by suspending 1 kg of fresh yeast (27-29% dry matter) into 240 ml of a 30% aqueous solution of partially hydrolysed starch.

### Example 11

### The viscosity of high-density liquid yeast preparations

The viscosity of a high-density liquid yeast is typically less than 200 centiPoise (cP) at 20°C. The viscosities at 20°C of conventional liquid yeast preparations and of different yeast preparations according to the invention are shown in greater detail in Figures 4 and 5.

Bars G1, G2 and G3 indicate the viscosities at 20°C of high-density yeasts that contain 1040 grams yeast (27-29% dry matter) per liter, the extracellular concentrations of glycerol being 14.1, 10.6 and 7.0% (w/v), respectively.

Bars G4, G5 and G6 indicate the viscosities of high-density yeasts that have been adjusted to contain 1000 grams yeast (27-29% dry matter) per liter, the extracellular concentrations of glycerol being 14.9, 11.1 and 7.4% (w/v), respectively.

Bars W1, W2 and W3 indicate the viscosities of conventional yeast creams in water with yeast contents of 750, 800 and 830 grams per liter, respectively.

Bars EG and PG indicate the viscosities of high-density yeasts that contain 1040 grams of yeast per liter, the extracellular concentrations of ethylene glycol and propylene glycol being 17.8 and 16.6% (w/v), respectively.

Bars S1 and S2 indicate the viscosities of high-density yeasts that contain 1000 and 960 grams yeast, the extracellular concentrations of sorbitol being 8.3 and 10.9% (w/v), respectively.

## Claims

1. A high-density liquid or pasty yeast preparation having a capability of retaining its activity, dissolving instantly and being easily batched, uniformly suspendable and tolerant of repeated freezing and thawing, and comprising 1-20% (w/w) of a polyhydroxy compound selected from a group comprising propylene glycol, glycerol, nonfermentable mono- or oligosaccharides such as xylose, or non-fermentable sugar alcohols such as mannitol and sorbitol, soluble oligo- or polymeric carbohydrates such as partially hydrolysed starch, cellulose or agarose or derivatives thereof and polyethylene glycol, or mixtures thereof, and 80-99% (w/w) of fresh yeast, said high-density yeast preparation having a density of more than 800 g yeast (27-29% dry matter) per 1 liter of the yeast preparation, the concentration of the polyhydroxy compound in the extracellular liquid being 5-50% (w/v).

2. A high-density liquid or pasty yeast preparation according to claim 1, wherein the polyhydroxy compound is glycerol and the yeast preparation comprises 10-200 g glycerol per 1 kg of fresh yeast, said high-density yeast preparation having a density of more than 800 g yeast (27-29% dry matter) per 1 liter of the preparation, the glycerol concentration in the extracellular liquid being 5-50% (w/v).

3. A high-density liquid yeast preparation having a capability of retaining its activity, dissolving instantly and being easily batched, uniformly suspendable and tolerant of repeated freezing and thawing, and comprising 3-20% (w/w) of a polyhydroxy compound selected from a group comprising propylene glycol, glycerol, non-fermentable mono- or oligosaccharides such as xylose, or non-fermentable sugar alcohols such as mannitol and sorbitol, soluble oligo- or polymeric carbohydrates such as partially hydrolysed starch, cellulose or agarose or derivatives thereof and polyethylene glycol, or mixtures thereof, and 80-97% of fresh yeast, said high-density yeast preparation having a density of more than 800 g yeast (27-29% dry matter) per 1 liter of the preparation, the concentration of the polyhydroxy compound in the extracellular liquid being 10-50, preferably 20% (w/v), and said yeast preparation having a viscosity of less than 200 centiPoise (cP) at 20°C.

4. A high-density liquid yeast preparation according to claim 3, wherein the polyhydroxy compound is glycerol and the yeast preparation comprises 30-200 g glycerol per 1 kg of fresh yeast, said high-density yeast preparation having a density of more than 800 g yeast (27-29% dry matter) per 1 liter of the preparation, the glycerol concentration in the extracellular liquid being 10-50, preferably 20% (w/v), and said yeast preparation having a viscosity of less than 200 centiPoise (cP) at 20°C.

5. A high-density pasty yeast preparation having a capability of retaining its activity, dissolving instantly and being easily batched, uniformly suspendable and tolerant of repeated freezing and thawing, and comprising 1-4% (w/w) of a polyhydroxy compound selected from a group comprising propylene glycol, glycerol, non-fermentable mono- or oligosaccharides such as xylose, or non-fermentable sugar alcohols such as mannitol and sorbitol, soluble oligo- or polymeric carbohydrates such as partially hydrolysed starch, cellulose or agarose or derivatives thereof and polyethylene glycol, or mixtures thereof, and 96-99% (w/w) of fresh yeast, said high-density yeast preparation having a density of more than 800 g yeast (27-29% dry matter) per 1 liter of the preparation, the concentration of the polyhydroxy compound in the extracellular liquid being 5-15, preferably 10% (w/v).

6. A high-density pasty yeast preparation according to claim 5, wherein the polyhydroxy compound is glycerol and the yeast preparation comprises 10-40 g glycerol per 1 kg of fresh yeast, said high-density yeast preparation having a density of more than 800 g yeast (27-29% dry matter) per 1 liter of the preparation, the glycerol concentration in the extracellular liquid being 5-15, preferably 10% (w/v).

7. A method for preparing a high-density yeast preparation according to any one of claims 1 to 6, comprising the addition of 1-20% (w/w) of a polyhydroxy compound selected from a group comprising propylene glycol, glycerol, non-fermentable mono- or oligosaccharides such as xylose, or non-fermentable sugar alcohols such as mannitol and sorbitol, soluble oligo- or polymeric carbohydrates such as partially hydrolysed starch, cellulose or agarose or derivatives thereof and polyethylene glycol or mixtures thereof to fresh yeast by means of gentle agitation.

8. A method for preparing the high-density yeast preparation according to any one of claims 2, 4 and 6, comprising the addition of 10-200 g of glycerol per 1 kg of fresh yeast by means of gentle agitation.

9. A method for preparing the high-density liquid yeast preparation according to claim 3 or 4, comprising the addition of 3-20% (w/w) of a polyhydroxy compound selected from a group comprising propylene glycol, glycerol, non-fermentable mono- or oligosaccharides such as xylose, or non-fermentable sugar alcohols such as mannitol and sorbitol, soluble oligo- or polymeric carbohydrates such as partially hydrolysed starch, cellulose or agarose or derivatives thereof and polyethylene glycol or mixtures thereof to fresh yeast by means of gentle agitation.

10. A method for preparing the high-density liquid yeast preparation according to claim 4, comprising the addition of 30-200 g of glycerol per 1 kg of fresh yeast by means of gentle agitation.

11. A method for preparing the high-density pasty yeast preparation according to claim 5 or 6, comprising the addition of 1-4% (w/w) of a polyhydroxy compound selected from a group comprising propylene glycol, glycerol, non-fermentable mono- or oligosaccharides such as xylose, or non-fermentable sugar alcohols such as mannitol and sorbitol, soluble oligo- or polymeric carbohydrates such as partially hydrolysed starch, cellulose or agarose or derivatives thereof and polyethylene glycol or mixtures thereof to fresh yeast by means of gentle agitation.

12. A method for preparing the high-density pasty yeast preparation according to claim 6, comprising the addition of 10-40 g of glycerol per 1 kg of fresh yeast by means of gentle agitation.

13. A high-density liquid yeast preparation according to claim 3 or 4 for substituting for cream yeast.

14. A high-density pasty yeast preparation according to claim 5 or 6 in an extrudable batching form.

15. A high-density liquid yeast preparation according to claim 3 or 4 in a single package containing more than one dosage and optionally provided with a dosaging device.

16. A high-density liquid or pasty yeast preparation according to any one of claims 1 to 6 in a combination package comprising a plurality of singlebatch containers.

17. A high-density baker's special yeast with improved quality prepared by the methods according to any one of claims 7 to 12.

18. A high-density sourdough yeast with improved quality prepared by the methods according to any one of claims 7 to 12 and having the properties defined in claims 1-6.

19. A high-density brewer's yeast with improved quality prepared by the methods according to any one of claims 7 to 12 and having the properties defined in claims 1-6.

20. A high-density baker's yeast with improved quality prepared by the methods according to any one of claims 7 to 12 and having the properties defined in claims 1-6.

21. A high-density wine yeast with improved quality prepared by the methods according to any one of claims 7 to 12 and having the properties defined in claims 1-6.

22. A high-density distiller's yeast with improved quality prepared by the methods according to any one of claims 7 to 12 and having the properties defined in claims 1-6.

## Patentansprüche

1. Hochdichtes flüssiges oder pastöses Hefepräparat mit Fähigkeit zum Erhalt seiner Aktivität, Instantlöslichkeit und leicht portionierbar, gleichmäßig suspendierbar und tolerant gegenüber wiederholtem Einfrieren und Auftauen und umfassend 1 - 20 % (Gew./Gew.) einer Polyhydroxyverbindung ausgewählt aus einer Gruppe umfassend Propylenglykol, Glycerin, nichtfermentierbare Mono- oder Oligosaccharide wie Xylose oder nichtfermentierbare Zuckeralkohole wie Mannit und Sorbit, lösliche oligomere oder polymere Kohlehydrate wie teilweise hydrolysierte Stärke, Cellulose oder Agarose oder Derivate davon und Polyethylenglykol oder Mischungen davon und 80 - 99 % (Gew./Gew.) frische Hefe, wobei das hochdichte Hefepräparat eine Dichte von mehr als 800 g Hefe (27 - 29 % Trockenmasse) auf 1 Liter des Hefepräparats aufweist, wobei die Konzentration der Polyhydroxyverbindung in der extrazellulären Flüssigkeit 5 - 50 % (Gew./Vol.) beträgt.

2. Hochdichtes flüssiges oder pastöses Hefepräparat nach Anspruch 1, worin die Polyhydroxyverbindung Glycerin ist und das Hefepräparat 10 - 200 g Glycerin auf 1 kg frische Hefe umfaßt, wobei das hochdichte Hefepräparat eine Dichte von mehr als 800 g Hefe (27 - 29 % Trockenmasse) auf 1 Liter des Präparats aufweist, wobei die Glycerinkonzentration in der extrazellulären Flüssigkeit 5 - 50 % (Gew./Vol.) beträgt.

3. Hochdichtes flüssiges Hefepräparat mit Fähigkeit zum Erhalt seiner Aktivität, Instantlöslichkeit und leicht portionierbar, gleichmäßig suspendierbar und tolerant gegenüber wiederholtem Einfrieren und Auftauen und umfassend 3 - 20 % (Gew./Gew.) einer Polyhydroxyverbindung ausgewählt aus einer Gruppe umfassend Propylenglykol, Glycerin, nichtfermentierbare Mono- oder Oligosaccharide wie Xylose oder nichtfermentierbare Zuckeralkohole wie Mannit und Sorbit, lösliche oligomere oder polymere Kohlehydrate wie teilweise hydrolysierte Stärke, Cellulose oder Agarose oder Derivate davon und Polyethylenglykol oder Mischungen davon und 80 - 97 % frische Hefe, wobei das hochdichte Hefepräparat eine Dichte von mehr als 800 g Hefe (27 - 29 % Trockenmasse) auf 1 Liter des Präparats aufweist, wobei die Konzentration der Polyhydroxyverbindung in der extrazellulären Flüssigkeit 10 - 50, bevorzugt 20 % (Gew./Vol.) beträgt und wobei das Hefepräparat eine Viskosität von weniger als 200 Centipoise (cP) bei 20 °C aufweist.

4. Hochdichtes flüssiges Hefepräparat nach Anspruch 3, worin die Polyhydroxyverbindung Glycerin ist und das Hefepräparat 30 - 200 g Glycerin auf 1 kg frische Hefe umfaßt, wobei das hochdichte Hefepräparat eine Dichte von mehr als 800 g Hefe (27 - 29 % Trockenmasse) auf 1 Liter des Präparats aufweist, wobei die Glycerinkonzentration in der extrazellulären Flüssigkeit 10 - 50, bevorzugt 20 % (Gew./Vol.) beträgt und wobei das Hefepräparat eine Viskosität von weniger als 200 Centipoise (cP) bei 20 °C aufweist.

5. Hochdichtes pastöses Hefepräparat mit Fähigkeit zum Erhalt seiner Aktivität, Instantlöslichkeit und leicht portionierbar, gleichmäßig suspendierbar und tolerant gegenüber wiederholtem Einfrieren und Auftauen und umfassend 1 - 4 % (Gew./Gew.) einer Polyhydroxyverbindung ausgewählt aus einer Gruppe umfassend Propylenglykol, Glycerin, nichtfermentierbare Mono- oder Oligosaccharide wie Xylose oder nichtfermentierbare Zuckeralkohole wie Mannit und Sorbit, lösliche oligomere oder polymere Kohlehydrate wie teilweise hydrolysierte Stärke, Cellulose oder Agarose oder Derivate davon und Polyethylenglykol oder Mischungen davon und 96 - 99 % (Gew./Gew.) frische Hefe, wobei das hochdichte Hefepräparat eine Dichte von mehr als 800 g Hefe (27 - 29 % Trockenmasse) auf 1 Liter des Präparats aufweist, wobei die Konzentration der Polyhydroxyverbindung in der extrazellulären Flüssigkeit 5 - 15 %, bevorzugt 10 % (Gew./Vol.) beträgt.

6. Hochdichtes pastöses Hefepräparat nach Anspruch 5, worin die Polyhydroxyverbindung Glycerin ist und das Hefepräparat 10 - 40 g Glycerin auf 1 kg frische Hefe umfaßt, wobei das hochdichte Hefepräparat eine Dichte von mehr als 800 g Hefe (27 - 29 % Trockenmasse) auf 1 Liter des Präparats aufweist, wobei die Glycerinkonzentration in der extrazellulären Flüssigkeit 5 - 15, bevorzugt 10 % (Gew./Vol.) beträgt.

7. Verfahren zur Herstellung eines hochdichten Hefepräparats nach einem der Ansprüche 1 bis 6, umfassend den Zusatz von 1 - 20 % (Gew./Gew.) einer Polyhydroxyverbindung ausgewählt aus einer Gruppe umfassend Propylenglykol, Glycerin, nichtfermentierbare Mono- oder Oligosaccharide wie Xylose oder nichtfermentierbare Zuckeralkohole wie Mannit und Sorbit, lösliche oligomere oder polymere Kohlehydrate wie teilweise hydrolysierte Stärke, Cellulose oder Agarose oder Derivate davon und Polyethylenglykol oder Mischungen davon zu frischer Hefe mittels schonender Vermischung.

8. Verfahren zur Herstellung des hochdichten Hefepräparats nach einem der Ansprüche 2, 4 und 6 umfassend den Zusatz von 10 - 200 g Glycerin auf 1 kg frische Hefe mittels schonender Vermischung.

9. Verfahren zur Herstellung des hochdichten flüssigen Hefepräparats nach Anspruch 3 oder 4 umfassend den Zusatz von 3 - 20 % (Gew./Gew.) einer Polyhydroxyverbindung ausgewählt aus einer Gruppe umfassend Propylenglykol, Glycerin, nichtfermentierbare Mono- oder Oligosaccharide wie Xylose oder nichtfermentierbare Zuckeralkohole wie Mannit und Sorbit, lösliche oligomere oder polymere Kohlehydrate wie teilweise hydrolysierte Stärke, Cellulose oder Agarose oder Derivate davon und Polyethylenglykol oder Mischungen davon zu frischer Hefe mittels schonender Vermischung.

10. Verfahren zur Herstellung des hochdichten flüssigen Hefepräparats nach Anspruch 4 umfassend den Zusatz von 30 - 200 g Glycerin auf 1 kg frische Hefe mittels schonender Vermischung.

11. Verfahren zur Herstellung des hochdichten pastösen Hefepräparats nach Anspruch 5 oder 6 umfassend den Zusatz von 1 - 4 % (Gew./Gew.) einer Polyhydroxyverbindung ausgewählt aus einer Gruppe umfassend Propylenglykol, Glycerin, nichtfermentierbare Mono- oder Oligosaccharide wie Xylose oder nichtfermentierbare Zuckeralkohole wie Mannit und Sorbit, lösliche oligomere oder polymere Kohlehydrate wie teilweise hydrolysierte Stärke, Cellulose oder Agarose oder Derivate davon und Polyethylenglykol oder Mischungen davon zu frischer Hefe mittels schonender Vermischung.

12. Verfahren zur Herstellung des hochdichten pastösen Hefepräparats nach Anspruch 6 umfassend den Zusatz von 10 - 40 g Glycerin auf 1 kg frische Hefe mittels schonender Vermischung.

13. Hochdichtes flüssiges Hefepräparat nach Anspruch 3 oder 4 als Ersatz für Cremehefe.

14. Hochdichtes pastöses Hefepräparat nach Anspruch 5 oder 6 in einer extrudierbaren Portionierform.

15. Hochdichtes flüssiges Hefepräparat nach Anspruch 3 oder 4 in einer Einzelpackung enthaltend mehr als eine Portion und gegebenenfalls versehen mit einer Dosiervorrichtung.

16. Hochdichtes flüssiges oder pastöses Hefepräparat nach einem der Ansprüche 1 bis 6 in einer Kombinationspackung umfassend eine Vielzahl von Einzelportionsbehältern.

17. Hochdichte Bäckerspezialhefe mit verbesserter Qualität hergestellt nach den Verfahren nach einem der Ansprüche 7 bis 12.

18. Hochdichte Sauerteighefe mit verbesserter Qualität hergestellt nach den Verfahren nach einem der Ansprüche 7 bis 12 und mit den Eigenschaften definiert in den Ansprüchen 1 bis 6.

19. Hochdichte Bierhefe mit verbesserter Qualität hergestellt nach den Verfahren nach einem der Ansprüche 7 bis 12 und mit den Eigenschaften definiert in den Ansprüchen 1 bis 6.

20. Hochdichte Bäckerhefe mit verbesserter Qualität hergestellt nach den Verfahren nach einem der Ansprüche 7 bis 12 und mit den Eigenschaften definiert in den Ansprüchen 1 bis 6.

21. Hochdichte Weinhefe mit verbesserter Qualität hergestellt nach den Verfahren nach einem der Ansprüche 7 bis 12 und mit den Eigenschaften definiert in den Ansprüchen 1 bis 6.

22. Hochdichte Branntweinhefe mit verbesserter Qualität hergestellt nach den Verfahren nach einem der Ansprüche 7 bis 12 und mit den Eigenschaften definiert in den Ansprüchen 1 bis 6.

## Revendications

1. Préparation liquide ou pâteuse de levure de haute densité ayant la capacité de retenir son activité, de solution immédiate et de dosage, la mise en suspension régulière et admettant la congélation et la décongélation et comportant 1-20% (par poids) de composé polyhydroxy sélectionné à partir d'un groupe comportant le propylène glycol, le glycérol, les mono- ou oligo-saccharides non-fermentables tels que le xylose, ou les alcools non fermentables de sucre tels que mannitol et sorbitol, oligo- ou carbohydrates polymériques solubles tels que l'amidon partiellement hydrolysé, la cellulose ou l'agarose ou leur dérivés et le glycol polyéthylène ou leurs mélanges, et 80-99% de levure fraîche, ladite préparation de levure de haute densité ayant une densité supérieure à 800 g de levure (27-29% de matières sèches) par litre de préparation de levure, la concentration du composé polyhydroxy en liquide extra-cellulaire étant de 5-50% (poids/volume).

2. Préparation liquide ou pâteuse de levure de haute densité selon la revendication 1, dont le composé polyhydroxy est le glycérol et la préparation de levure comporte 10-200 g de glycérol par kg de levure fraîche, ladite préparation de levure de haute densité ayant une densité supérieure à 800 g de levure (27-29% de matières sèches) par litre de préparation, la concentration de glycérol dans le liquide extra-cellulaire étant de 5-50% (poids/volume).

3. Préparation liquide ou pâteuse de levure de haute densité selon la revendication 1, ayant la capacité de retenir son activité, de solution immédiate et de dosage, la mise en suspension régulière et admettant la congélation et la décongélation et comportant 3-20% (par poids) de composé polyhydroxy sélectionné à partir d'un groupe comportant le propylène glycol, le glycérol, les mono- ou oligosaccharides non-fermentables tels que le xylose, ou les alcools non fermentables de sucre tels que mannitol et sorbitol, oligo- ou carbohydrates polymériques solubles tels que l'amidon partiellement hydrolysé, la cellulose ou l'agarose ou leur dérivés et le glycol polyéthylène ou leurs mélanges, et 80-97% de levure fraîche, ladite préparation de levure de haute densité ayant une densité supérieure à 800 g de levure (27-29% de matières sèches) par litre de préparation de levure, la concentration du composé polyhydroxy en liquide extra-cellulaire étant de 10-50, préférablement 20% (poids/volume) et ladite préparation de levure ayant une viscosité inférieure à 200 centiPoises (cP) à 20°C.

4. Préparation liquide de levure de haute densité selon la revendication 3, dont le composé polyhydroxy est le glycérol, et la préparation de levure comporte 30-200 g de glycérol par kg de levure fraîche, ladite préparation de levure de haute densité comportant une densité supérieure à 800 g de levure (27-29% de matières sèches) par litre de préparation, la concentration du glycérol en liquide extra-cellulaire étant de 10-50, préférablement 20% (poids/volume) et ladite préparation de levure ayant une viscosité inférieure à 200 centiPoises (cP) à 20°C.

5. Préparation pâteuse de levure de haute densité ayant la capacité de retenir son activité, de solution immédiate et de dosage facile, admettant la mise en suspension régulière et la répétition de la congélation et la décongélation et comportant 1-4% (par poids) de composé polyhydroxy sélectionné à partir d'un groupe comportant le propylène glycol, le glycérol, les mono- ou oligo-saccharides non-fermentables tels que le xylose, ou les alcools non fermentables de sucre tels que mannitol et sorbitol, oligo- ou carbohydrates polymériques solubles tels que l'amidon partiellement hydrolysé, la cellulose ou l'agarose ou leur dérivés et le glycol polyéthylène ou leurs mélanges, et 96-99% (par poids) de levure fraîche, ladite préparation de levure de haute densité ayant une densité supérieure à 800 g de levure (27-29% de matières sèches) par litre de préparation, la concentration du composé polyhydroxy en liquide extra-cellulaire étant de 5-15, préférablement 10% (poids/volume).

6. Préparation pâteuse de levure de haute densité selon la revendication 5, dont le composé polyhydroxy est le glycérol, et ladite préparation de levure de haute densité comporte 10-40 g de glycérol par kg de levure fraîche, ladite préparation de levure de haute densité ayant une densité supérieure à 800 g de levure (27-29% de matières sèches) par litre de préparation, la concentration du glycérol en liquide extra-cellulaire étant de 5-15, préférablement 10% (poids/volume).

7. Méthode de préparation d'un composé de levure de haute densité selon l'une ou l'autre des revendications 1 à 6, comportant 1-20% (par poids) de composé polyhydroxy sélectionné à partir d'un groupe comportant le propylène glycol, le glycérol, les mono- ou oligosaccharides non-fermentables tels que le xylose, ou les alcools non fermentables de sucre tels que mannitol et sorbitol, oligo- ou carbohydrates polymériques solubles tels que l'amidon partiellement hydrolysé, la cellulose ou l'agarose ou leur dérivés et le glycol polyéthylène ou leurs mélanges, ajoutés à la levure fraîche et agités doucement.

8. Méthode de préparation d'un composé de levure de haute densité selon l'une ou l'autre des revendications 2, 4 et 6, comportant 10-200g de glycérol par kg de levure fraîche et agités doucement.

9. Méthode de préparation du composé liquide de levure de haute densité selon la revendication 3 ou 4, comportant 3-20% (par poids) de composé polyhydroxy sélectionné à partir d'un groupe comportant le propylène glycol, le glycérol, les mono- ou oligo-saccharides non-fermentables tels que le xylose, ou les alcools non fermentables de sucre tels que mannitol et sorbitol, oligo- ou carbohydrates polymériques solubles tels que l'amidon partiellement hydrolysé, la cellulose ou l'agarose ou leur dérivés et le glycol polyéthylène ou leurs mélanges, ajoutés à la levure fraîche et agités doucement.

10. Méthode de préparation d'un composé liquide de levure de haute densité selon la revendication 4, comportant l'addition de 30-200g de glycérol par kg de levure fraîche et agités doucement.

11. Méthode de préparation du composé pâteux de levure de haute densité selon la revendication 5 ou 6, comportant 1-4% (par poids) de composé polyhydroxy sélectionné à partir d'un groupe comportant le propylène glycol, le glycérol, les mono- ou oligo-saccharides non-fermentables tels que le xylose, ou les alcools non fermentables de sucre tels que mannitol et sorbitol, oligo- ou carbohydrates polymériques solubles tels que l'amidon partiellement hydrolysé, la cellulose ou l'agarose ou leur dérivés et le glycol polyéthylène ou leurs mélanges, ajoutés à la levure fraîche et agités doucement.

12. Méthode de préparation d'un composé pâteux de levure de haute densité selon la revendication 6, comportant l'addition de 10-40g de glycérol par kg de levure fraîche et agités doucement.

13. Préparation d'un composé liquide de levure de haute densité selon la revendication 3 ou 4 pour remplacer la crème de levure.

14. Préparation d'un composé pâteux de levure de haute densité selon la revendication 5 ou 6 sous forme dosable en extrusion.

15. Préparation liquide de levure de haute densité selon la revendication 3 ou 4 en conditionnement unique comportant plusieurs doses et prévoyant un doseur en option.

16. Préparation liquide ou pâteuse de levure de haute densité selon l'une ou l'autre des revendications 1 à 6 en conditionnement combiné comportant une pluralité de récipients pour dose unique.

17. Levure spéciale de haute densité pour la boulangerie, de qualité améliorée préparée selon l'une ou l'autre des méthodes aux revendications 7 à 12.

18. Levure de levain de haute densité de qualité perfectionnée préparée suivant les méthodes selon l'une ou l'autre des revendications 7 à 12, et ayant les caractéristiques indiquées aux revendications 1-6.

19. Levure de brasserie de haute densité de qualité perfectionnée préparée suivant les méthodes selon l'une ou l'autre des revendications 7 à 12, et ayant les caractéristiques indiquées aux revendications 1-6.

20. Levure de boulangerie de haute densité de qualité perfectionnée préparée suivant les méthodes selon l'une ou l'autre des revendications 7 à 12, et ayant les caractéristiques indiquées aux revendications 1-6.

21. Levure de vin de haute densité de qualité perfectionnée préparée suivant les méthodes selon l'une ou l'autre des revendications 7 à 12, et ayant les caractéristiques indiquées aux revendications 1-6.

22. Levure de distillerie de haute densité de qualité perfectionnée préparée suivant les méthodes selon l'une ou l'autre des revendications 7 à 12, et ayant les caractéristiques indiquées aux revendications 1-6.
